# FASCICULE DE BREVET EUROPEEN

(11) **EP 1 165 052 B1**
(45) Date de publication et mention de la délivrance du brevet: **09.06.2004**
(21) Numéro de dépôt: 00915253.9
(22) Date de dépôt: 30.03.2000
(51) Int. Cl.: A61K 9/20

(54) **COMPOSITION OU STRUCTURE MICROPOREUSE EXPANSEE ISOTROPE A DISSOLUTION RAPIDE A USAGE PHARMACEUTIQUE, VETERINAIRE, DIETETIQUE, ALIMENTAIRE OU COSMETIQUE ET SON PROCEDE D'OBTENTION**
ZUSAMMENSETZUNG ODER EXPANDIERTE MIKROPORÖSE STRUKTUR MIT RASCHER AUFLÖSUNG , ZUR VERWENDUNG IM PHARMAZEUTISCHEN, VETERINÄRMEDIZINISCHEN, DIÄTETISCHEN, KOSMETISCHEN UND LEBENSMITTEL-BEREICH UND VERFAHREN ZU SEINER HERSTELLUNG
FAST-DISSOLVING ISOTROPIC EXPANDED MICROPOROUS COMPOSITION OR STRUCTURE FOR PHARMACEUTICAL, VETERINARY, DIETETIC, FOOD OR COSMETIC USE AND METHOD FOR OBTAINING THE SAME

(30) Priorité: 31.03.1999 FR 9904033
(43) Date de publication de la demande: 02.01.2002
(73) Titulaire: PIERRE FABRE MEDICAMENT, 92100 Boulogne (FR)
(72) Inventeur: GOUTAY, Eric, F-31650 Lauzerville (FR); LACHAMP, Laurence, F-31650 St Orens de Gameville (FR); FRANCES, Jacques, F-31300 Toulouse (FR); BOUGARET, Jo[l, F-31570 Lanta (FR); PAILLARD, Bruno, F-31650 St Orens de Gameville (FR)
(74) Mandataire: Ahner, Francis
(86) Numéro de dépôt international: PCT/FR2000/000803
(87) Numéro de publication internationale: WO 2000/057856

(56) Documents cités:
- EP-A- 0 147 335
- EP-A- 0 549 420
- WO-A-91/09591
- GB-A- 652 901
- DATABASE WPI Section Ch, Week 198141 Derwent Publications Ltd., London, GB; Class A85, AN 1981-74678D XP002126187 & JP 56 108523 A (NEW NIPPON ELECTRIC CO LTD), 28 août 1981 (1981-08-28)

## Description

L'invention concerne de nouvelles compositions microporeuses homogènes à usage pharmaceutique, vétérinaire, alimentaire, diététique ou cosmétique à délitement rapide, voire instantané, destinées à la voie orale ou à être appliquées au contact de muqueuses et leur procédé d'obtention.

Les compositions solides à délitement rapide ou instantané pour la voie orale ont depuis très longtemps intéressé le formulateur mais également le praticien et le patient qui trouvèrent auprès de ces dernières, des caractéristiques intéressantes en terme d'observance. Concernant les sujets très jeunes ou âgés chez qui la déglutition des formes solides posent problème, les compositions telles que présentées dans la présente invention apportent un réel avantage car elles peuvent être prises soit dans un verre d'eau ou soit directement sous la langue ou elles se délitent instantanément.

De part ces caractéristiques, les compositions faisant l'objet de l'invention représentent la solution idéale pour un traitement ambulatoire.

De plus, elles répondent favorablement à l'association inconsciente faite par le patient entre rapidité de dissolution ou de délitement de la composition et rapidité d'action de la molécule, notamment pour les antalgiques, les antinauséeux, les antiulcéreux, les antiasthmatiques et les anti-angineux. Cette association inconsciente pouvant dans quelques cas améliorer l'efficacité de la molécule.

On entend par forme à délitement rapide des formes galéniques dont le délitement reste inférieur à 15 minutes en accord avec la monographie comprimés (Compressi) de la pharmacopée française ou européenne.

Dans le domaine de la pharmacie plusieurs formulations à délitement rapide sont déjà utilisées. Les comprimés ou granulés effervescents permettent le délitement en moins de 5 minutes par la mise en solution ou la dispersion rapide de la molécule grâce à un dégagement de gaz carbonique contrôlé et obtenu à partir d'une réaction chimique acide - base.

Cette technologie, très utilisée aujourd'hui et décrite dans de nombreux brevets (EP 673 644 ; EP 369 228 ; FR 2 552 308), reste maîtrisée au niveau industriel par peu de sociétés. En effet, cette technique nécessite un savoir-faire important dans la conduite de l'étape de granulation humide, mais également un environnement à humidité contrôlée très coûteux à entretenir.

De plus, la taille et l'effervescence importantes de la forme, ne permettent pas d'utiliser ces comprimés effervescents conventionnels, dans la cavité buccale ou en l'absence d'eau.

Ce problème a été résolu dans les nouvelles formulations appelées micro-effervescentes qui ont fait l'objet du brevet américain récent US 5 178 878.

Les comprimés ou granulés hydrodispersibles constituent des formes à délitement rapide dont la propriété repose essentiellement sur l'emploi de composés appelés superdélitants. Au contact de l'eau, ces derniers produisent, par leur pouvoir gonflant très important, « l'explosion » de la masse comprimée ou granulaire.

De nombreux brevets décrivent ce type de formes galéniques (FR 95/00947, EP 0 347 767, EP 0 716 852 et EP 0 361 354) et la grande majorité utilise les composés suivants : glycolate d'amidon, cellulose microcristalline, carboxyméthylcellulose et polyvinylpyrrolidone réticulées.

Quelques auteurs emploient des délitants moins communs tels que les argiles type smectite ou actapulgite (WO 92/13527), ou les gommes et plus particulièrement la gomme guar (EP 0 273 005).

Comme pour les comprimés effervescents, ces formes sont très difficilement utilisables sans eau et donc mal adaptées à une utilisation buccale ou sublinguale ambulatoire. Il est également nécessaire, dans de très nombreux cas, d'augmenter le volume et ainsi le poids du comprimé afin d'avoir une surface spécifique compatible avec un délitement rapide.

La formulation de ce type de comprimés qui peut paraître simple à première vue, est en fait assez complexe et repose sur un compromis entre la dureté et le délitement qu'il faut optimiser au mieux, en fonction de la nature physico-chimique et du taux de principe actif.

Dernièrement, le brevet EP 764 019 décrit la mise au point à partir de sucres amorphisés par filage, des formes à délitement rapide par un procédé minimisant la phase de compression (compaction par compressodoseur). Compte tenu de la faible dureté des compacts, la société détentrice de cette nouvelle forme, a dû résoudre l'étape de mise en conditionnement (type blister) par l'adaptation de procédés peu compatibles avec des cadences industrielles.

De plus, les technologies comprimés effervescents et hydrodispersibles reposent sur des procédés discontinus incluant une phase de compression d'un ou de mélange(s) pulvérulent(s).

Cela entraîne nécessairement une faible cadence de production par rapport à un procédé en continu et, par conséquence, une augmentation du prix de revient.

Parallèlement, aux deux formes de comprimés précédentes, il existe dans le domaine de la pharmacie des formes unitaires solides fabriquées par lyophilisation appelées lyophilisats oraux.

Cette technologie de lyophilisation est connue depuis des années (FR 2 403 078) et utilisée pour conserver et administrer les molécules sensibles au plan physico-chimique.

Cette technologie lourde et coûteuse dont la durée de lyophilisation au niveau industriel est proche de 24 heures, dont la consommation énergétique est élevée (5 kW/h par kg d'eau) ne permet par opposition à la présente invention, d'être appliquée pour des raisons économiques à tous les produits.

Cependant, par l'emploi d'excipients judicieusement choisis, la lyophilisation permet d'aboutir à des formes présentant des délitements rapides soit au contact d'un volume convenable d'eau ou après mise au contact de la salive.

De nombreux documents récents décrivent ce type de formes galéniques (GB 2 111 423, US 5 039 540, US 5 120 549, WO 94 14422 et EP 651 997, EP 399 902).

Avantageusement, ces lyophilisats sont adaptés à une utilisation buccale et sublinguale ambulatoire. En revanche, lors de la mise au contact de la muqueuse buccale, les poudres solides utilisées dans la formulation confèrent une désagréable sensation granuleuse nettement perceptible. De plus, quelles que soient les formes à délitement rapide utilisées, leur mode de préparation délicat et peu flexible ne permet pas d'adapter la vitesse de délitement selon la nécessité d'utilisation.

L'objet de la présente invention est de proposer de nouvelles compositions et leur procédé d'obtention tel que décrit ci-après et illustré dans les exemples, permettant d'obtenir des temps de délitement égaux, voire inférieurs aux lyophilisats oraux. Comme ces derniers, la nouvelle forme pourra être dissoute, soit avec un volume d'eau adapté, soit directement dans la bouche ou au contact des muqueuses.

En revanche, les compositions selon l'invention grâce à leur formulation et leur procédé d'obtention en continu comportant une phase de mélange des composants, d'extrusion ou injection de la composition pâteuse dans un blister, puis une phase de séchage-formage (ou mise en forme) micro-ondes sous vide en continu, présentent une texture totalement différente où les particules solides solubilisées à un moment du procédé, ne sont plus perceptibles lors de la mise au contact de la muqueuse buccale. De plus, le procédé d'obtention en continu au niveau pilote ou industriel permet par son adaptabilité (temps fonction du volume) et sa moindre consommation énergétique d'être beaucoup moins onéreux que le procédé de lyophilisation

La composition selon l'invention à usage pharmaceutique, vétérinaire, alimentaire, diététique ou cosmétique et à dissolution rapide en milieu aqueux ou au contact des muqueuses comprend 1 % à 50 % en poids d'un ou plusieurs principe(s) actif(s), 50 % à 99 % en poids d'un support comprenant un ou plusieurs polymères, éventuellement un ou plusieurs diluant(s) et éventuellement un ou plusieurs additif(s), notamment arôme ou colorant, ladite composition étant caractérisée en ce qu'elle présente une structure expansée microporeuse isotrope à dissolution rapide et les polymères étant choisis dans le groupe constitué par les polymères d'origine végétale, éventuellement en combinaison avec les polymères d'origine animale ou les polymères de synthèse, et ledit support étant tel que le ou les polymère(s) liants sont présents dans la composition dans une proportion supérieure ou égale à 1% (p/p) et plus particulièrement comprise entre 6 % et 98 % (p/p).

La composition présente une structure poreuse, notamment une masse volumique inférieure à 0.9 g/cm³.

Un test de délitement approprié car illustrant le comportement en délitement des compositions consiste à placer la composition dans un bécher contenant 100 ml d'eau dont la température est comprise entre 15 et 25 °C. Le temps nécessaire pour que la totalité de la forme soit dissoute est noté.

D'autres part, la méthode USPXXIII appareil n° 2 dit appareil à palettes employant comme milieu de dissolution de l'eau distillée à 37 °C et une vitesse de rotation des palettes de 50 RPM a été utilisée comme test de dissolution in vitro.

Dans le cas de forme dite expansée, le taux d'expansion se réfère au rapport de volume des compositions après séchage-formage (ou mise en forme) par rapport au volume avant séchage.

Ce changement de volume s'accompagnant aussi d'une variation de la masse volumique.

Cette nouvelle forme pharmaceutique, vétérinaire, diététique, alimentaire ou cosmétique dont l'expansion, homogène et contrôlée du polymère grâce aux conditions opératoires de la phase de séchage-formage (ou mise en forme) micro-ondes sous vide permet d'obtenir une structure poreuse isotrope conférant alors une vitesse de délitement dans l'eau ou la cavité buccale ou au contact des muqueuses, pouvant aller de quelques secondes à plusieurs minutes selon la nécessité d'utilisation.

L'originalité de cette invention repose également sur le choix du ou des polymères, du ou des diluants utilisés pour la constitution du réseau matriciel de la forme mais également sur le procédé d'obtention qui permet d'obtenir en continu dans un temps inférieur à 1 heure préférentiellement inférieur à 30 minutes des formes dont on peut moduler la porosité et la forme durant la phase de séchage-formage (ou mise en forme) par micro-ondes sous vides en continu.

Parmi les principes actifs convenant pour réaliser la composition selon l'invention, on cite à titre indicatif et non limitatif les principes actifs choisis dans le groupe constitué par les médicaments ou les additifs alimentaires.

Les principes actifs utilisés présentent une solubilité très différente tels que le Milnacipran (solubilité aqueuse égale à 800 g/l), le piroxicam et la dompéridone (solubilité aqueuse inférieure à 100 mg/l ou le phloroglucinol (solubilité aqueuse voisine de 30 g/l).

On cite également à titre non limitatif, comme analgésiques anti-migraineux, les dérivés de l'ergot de seigle (ergotamine, dihydroergotamine, méthysergide) ou les antagonistes de la sérotonine (cyproheptadine, pizotifène, oxétérone). Comme analgésiques antipyrétiques et/ou anti-inflammatoires dérivés des arylcarboxyliques on cite l'acide salicylique, l'acide acétylsalicylique, l'acide méfénamique. Comme analgésique antipyrétiques et/ou anti-inflammatoires dérivés des acides arylalcanoïques, on cite le diclofenac, l'indométacine et comme dérivés analgésiques antipyrétiques et/ou anti-inflammatoires des acides énoliques, on cite la phénylbutazone et le ténoxicam. Comme anesthésiques locaux, on cite la lidocaïne et la tétracaïne. Comme anti-angineux on cite l'isosorbide 5-mononitrate, la molsidomine. Comme antispasmodiques anti-cholinergiques, on cite la métoclopramide, le lopéramide, la mébévérine, la papavérine, la trimébutine. Comme anti-secrétoires, on cite la cimétidine, la ranitidine. Comme myorelaxants, on cite le diazépam, le progabide, le dantrolène, le méphénésine, le baclofènen, les antiulcéreux (au sens large), les antihypertenseurs, les inhibiteurs enzyme de conversion, les antagonistes angiotensine II, les antagonistes du calcium β-bloquants, les vasodilatateurs périphériques centraux, les vasodilatateurs coronariens, les antiarthmiques, les antiagrégants plaquettaires, les antibiotiques, les corticoïdes oraux, les antimigraineux, les antipsychotiques, les hypnotiques, les sédatifs et les antinauséeux.

Le polymère selon l'invention, doit répondre à deux conditions souvent contradictoires, à savoir d'une part son caractère liant lui permettant d'être extrudé ou injecté puis mis en forme et, d'autre part sa capacité de délitement instantané après avoir été soumis au procédé de séchage-formage (ou mise en forme).

Les propriétés physico-chimiques, la concentration particulière peu élevée pour des formes à délitement rapide du ou des polymères matriciels ainsi que les conditions de séchage-formage (ou mise en forme) sont des critères importants car ils influencent fortement la porosité et la mise en forme par expansion de la forme et donc la vitesse de délitement, imposant donc un choix rigoureux de ces polymères au plan de la structure chimique et de la masse moléculaire mais également, un contrôle précis des paramètres de vides et d'énergie thermique utilisés, pour la mise en oeuvre de l'invention.

En effet, certains polymères, par leur caractère hydrophobe trop accentué, ne pourront convenir car quelle que soit leur masse moléculaire, ils ne pourront être dispersés et formulés en milieu aqueux dans une gamme de viscosité permettant leur répartition par injection ou extrusion. D'autres polymères hydrophiles de poids moléculaire trop élevé ou trop sensible à une élévation de température ne permettent pas non plus d'atteindre l'objectif selon l'invention.

A l'inverse le mauvais contrôle des conditions opératoires de séchage-formage (ou mise en forme) (vide, énergie thermique, durée) conduit selon la formulation à des formes non poreuses ou de porosité hétérogène ou à des structures trop expansées incompatibles avec l'utilisation selon l'invention.

Ces critères varieront selon le type de polymères ou l'association de polymères choisis.

Néanmoins, on a constaté en général que le polymère hydrophile devait se trouver dans un intervalle de masse moléculaire moyenne entre environ 1000 et 2 000 000 Da étant entendu que pour chaque polymère un sous-intervalle de masse moléculaire pourra être aisément déterminé par l'homme du métier, notamment par les tests de délitement indiqués ci-dessus.

Parmi ces polymères, on cite notamment les polysaccharides d'origine végétale obtenus par hydrolyse chimique ou enzymatique à partir des amidons natifs. Parmi les polysaccharides d'origine végétale obtenus par hydrolyse chimique ou enzymatique à partir de l'amidon natif, on cite notamment ceux qui répondent à la définition de maltodextrine ou de sirop de glucose. De préférence, le polymère d'origine végétale de type polysaccharide obtenu par hydrolyse chimique ou enzymatique est choisi parmi les maltodextrines ou sirops de glucose de degrés dextrose équivalent (DE) compris entre 3 et 50 et préférentiellement entre 6 et 34 ou leurs mélanges.

On cite également les polysaccharides d'origine végétale modifiés chimiquement. Par amidon modifié chimiquement, on entend le glycolate sodique d'amidon. Parmi les polymères hydrophiles on citera également les polymères chimiquement modifiés issus de la cellulose, les alkylcelluloses tels que l'hydroxypropyl cellulose (HPC), l'hydroxypropylméthylcellulose (HPMC), l'hydroxyethylcellulose, la carboxyméthylcellulose sodique (CMCNa) basse ou moyenne viscosité.

On cite aussi les polymères de type gomme. Comme polymère de type gomme, on cite le guar, la gomme arabique, la gomme xanthane, la pectine et les alginates ou leurs mélanges.

Parmi les polymères de synthèse, on cite les polyéthylène glycols (PEG), la polyvinylpyrrolidone (PVP).

Parmi les polymères d'origine animale, on cite les protéines telles que la gélatine, le collagène, les caséinates sodiques, l'acide chondroïtique sulfate et leurs hydrolysats, les chitosans et leurs dérivés d'hydrolyse solubles ou leurs mélanges.

Les mélanges de ces différents polymères dans les proportions appropriées sont également envisagés. En effet, par exemple dans le cas d'un mélange maltodextrine/PVP, il y a formation de structures microporeuses très solubles.

De préférence, le ou lesdits polymères sont présents dans la formulation à un pourcentage compatible avec une viscosité comprise entre 100 mPa.s et 100 000 mPa.s, de préférence entre 100 et 50 000 mPa.s.

Parmi les diluants on cite le mannitol, le saccharose, le lactose, le fructose, le sorbitol, le xylitol, le maltitol et le phosphate dicalcique dihydraté.

La composition selon l'invention peut comprendre jusqu'à 10 % d'additifs. Ces additifs sont notamment choisis dans le groupe constitué par les plastifiants, les arômes, les colorants, les opacifiants.

De préférence, la composition à usage pharmaceutique ou alimentaire selon l'invention présente un temps de délitement compris entre 1 seconde et 10 minutes, de préférence inférieur à 1 minute, avantageusement inférieur à 30 secondes, lors de la prise par le patient que ce soit en présence d'un volume adapté d'eau ou au contact direct de la muqueuse buccale ou toute autre muqueuse sur laquelle est appliquée la forme expansée microporeuse.

On peut également selon une variante avantageuse caractériser la composition par sa masse volumique, de préférence comprise entre 0,1 et 0,9 g/cm³, avantageusement entre 0,2 et 0,7 g/cm³.

En outre, la composition selon l'invention est telle que le ou les principe(s) actif(s) dans la matrice microporeuse ou poreuse expansée sont à l'état dissous, dispersé(s) ou sous formes pelliculées.

Selon un mode de réalisation avantageux le conditionnement final est du polypropylène ou du polytétrafluorothylène (Téflon® ).

L'invention concerne également un procédé de préparation des compositions selon l'invention comprenant le mélange du principe actif, des diluants et polymères et des additifs puis l'extrusion, ou l'injection directe dans un moule ou blister selon la viscosité de la formulation, ce moule ou blister et le procédé de séchage permettent de donner la forme finale à la composition.

Cette composition dite compacte est soumise à un traitement diélectrique continu micro-ondes sous vide instantané, assurant à la fois et de façon optimale, le séchage de la forme et la création de porosité et la mise en forme (ou formage) tout en évitant d'atteindre des niveaux thermiques trop élevés pouvant induire une dégradation du principe actif

La composition est ensuite récupérée et conditionnée de préférence dans le cadre d'un procédé en continu.

Selon un procédé général de mise en oeuvre, le procédé de préparation d'une composition à usage pharmaceutique, vétérinaire, alimentaire, diététique ou cosmétique, à délitement rapide l'invention est caractérisé en ce que l'on homogénéise une formulation pâteuse comprenant un ou plusieurs principes actifs, un ou plusieurs polymères, un ou plusieurs diluants et éventuellement un ou plusieurs additifs, on l'injecte dans un blister, puis en ce que l'on sèche-expanse et moule la forme par un procédé de type micro-ondes sous-vide, pour donner naissance à une structure microporeuse expansée isotrope, notamment de masse volumique inférieure à 0,9 g/cm³.

De préférence le procédé de préparation d'une composition à usage pharmaceutique ou alimentaire à délitement rapide est caractérisé en ce que le séchage-formage (ou mise en forme) et contrôle de la porosité se déroulent au cours d'une opération simultanée et est tel que le niveau de vide utilisé est compris de 30 à 700.10² Pa et préférentiellement compris entre 60 et 500.10² Pa (30 à 700 mbar et préférentiellement compris entre 60 et 500 mbar) pour donner naissance à une structure microporeuse expansée isotrope de forme régulière, notamment de masse volumique inférieure à 0,9 g/cm³.

Avantageusement, le procédé de préparation d'une composition à usage pharmaceutique, vétérinaire, alimentaire, diététique ou cosmétique microporeuse à délitement rapide est caractérisé en ce que la formulation pâteuse obtenue par homogénéisation présente une viscosité comprise entre 100 mPa.s et 100 000 mPa.s, préférentiellement entre 100 et 50 000 mPa.s, puis injection ou extrusion de cette masse dans un blister qui peut être avantageusement le conditionnement final. De préférence, les températures pendant la phase de séchage et mise en forme sont comprises entre 25 °C et 80 °C évitant de ce fait la dégradation des principes actifs thermolabiles.

La durée de l'opération de séchage et mise en forme est avantageusement inférieure à 1 heure préférentiellement 30 minutes.

Selon la variation avantageuse, le blister est le conditionnement final de nature chimique de type polypropylène ou polytétrafluoroéthylène.

L'invention va maintenant être illustrée à titre non limitatif par les exemples suivants:

### Exemple n° 1

Un mélange (MD1) composé de 40 % d'eau, de 56 % de Maltodextrine de DE voisin de 19 et 4% d'arôme orange dont la viscosité est voisine de 600 mPa.s est réparti (environ 0,7 à 1 ml) dans des blisters polypropylène.

Ces échantillons sont introduits les uns après les autres, dans un four micro-ondes, relié à une pompe à vide et, soumis à différentes conditions opératoires.

Le procédé est ainsi conduit et contrôlé en continu par la maîtrise des niveaux énergétiques appliqués sur l'échantillon, de la température du produit et du niveau de vide appliqués sur l'échantillon.

### Condition opératoire a :

L'échantillon est injecté dans son blister polypropylène puis soumis à un niveau de vide de 20.10² Pa (20 mbar) et une puissance micro-ondes telle que l'échantillon absorbe environ 11W au cours des 10 minutes du procédé.

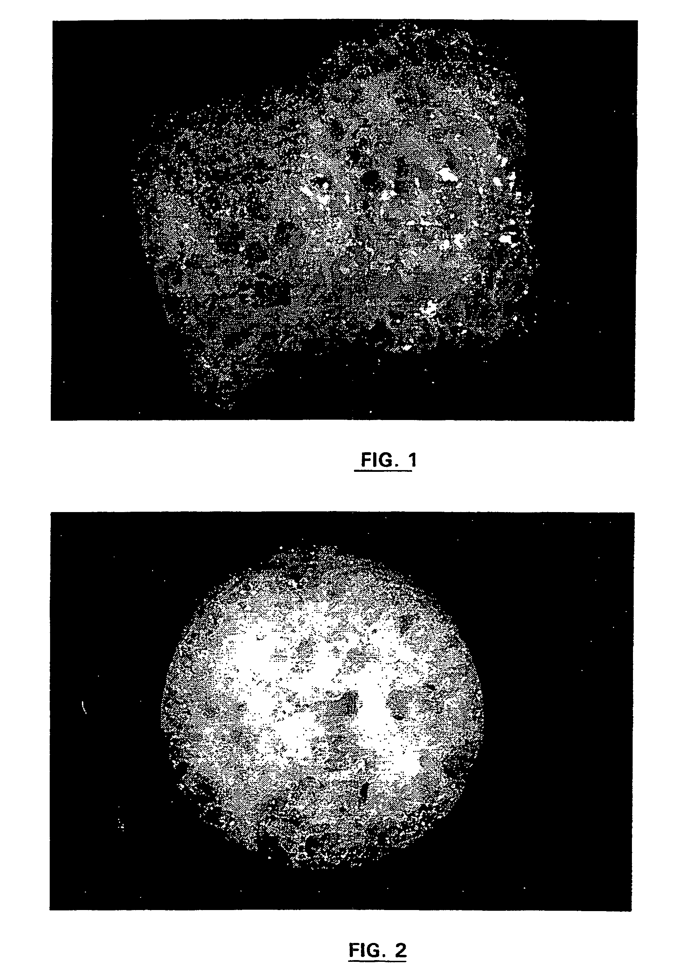

Dans ces conditions expérimentales (1a), l'échantillon subit très rapidement une expansion non contrôlée et un séchage, conduisant à une forme macroporeuse expansée non isotrope qualifiée de soufflée comme illustré sur la photographie de la Figure 1 avec un facteur d'agrandissement de 4, incompatible avec une utilisation dans le domaine pharmaceutique ou alimentaire.

### Condition opératoire b :

Un nouvel échantillon (0.7 ml) est injecté dans son blister polypropylène et est soumis pendant 15 minutes aux micro-ondes avec un niveau de pression de 60.10² Pa (60 mbar).

Dans ces conditions expérimentales (1b), l'échantillon absorbe entre 3 et 4 W et subit une expansion contrôlée et un séchage, conduisant à une forme expansée microporeuse isotrope de densité voisine de 0.22 et de volume voisin de 3 cm³, en accord avec l'objectif aux plans de la morphologie et du délitement. Un exemple des formes obtenues dans ces conditions sur la photographie en figure 2 (agrandissement de facteur 4).

En effet, les échantillons fabriqués selon ces conditions expérimentales présentent des délitements de 30 secondes dans un verre d'eau et de l'ordre d'une dizaine de seconde en bouche.

### Condition opératoire c :

Un nouvel échantillon (1c) est injecté dans son blister polypropylène et est soumis pendant 20 minutes, à une exposition de puissance telle qu'il absorbe 2.5 W et un niveau de vide de 90.10² Pa (90 mbar).

Dans ces conditions expérimentales (1c), l'échantillon subit une expansion contrôlée et un séchage, conduisant à une forme expansée microporeuse isotrope de densité voisine de 0.22 en accord avec l'objectif aux plans de la morphologie et du délitement.

En effet les échantillons fabriqués selon ces conditions expérimentales présentent des délitements de 30 secondes dans un verre d'eau et de l'ordre d'une dizaine de secondes en bouche.

### Condition opératoire d :

Un nouvel échantillon (1d) est injecté dans son blister polypropylène et est soumis pendant 15 minutes, à une exposition de puissance telle qu'il absorbe environ 3.5 W et un niveau de vide de 90.10² Pa (90 mbar) pendant 5 minutes puis 60.10² Pa (60 mbar) pendant 10 minutes.

Dans ces conditions expérimentales (1d), l'échantillon subit une expansion contrôlée et un séchage, conduisant à une forme expansée microporeuse isotrope de densité voisine de 0.2 en accord avec l'objectif aux plans de la morphologie et du délitement.

En effet les échantillons fabriqués selon ces conditions expérimentales présentent des délitements de 35 secondes dans 100 ml d'eau et de l'ordre d'une dizaine de seconde en bouche.

Cet exemple illustre parfaitement l'invention au plan de son procédé en ce sens qu'une même formule de base, soumise à différentes conditions de séchage micro-ondes sous vide aboutie à des formes expansées microporeuses isotropes à dissolution rapide de porosité et régularité dimensionnelle totalement différentes et maîtrisables.

En effet, le procédé de séchage selon l'invention permet de façon surprenante par un choix et un suivi judicieux des conditions opératoires niveau de vide et température du produit de gérer le séchage, la création de porosité et la mise en forme du produit fini.

Dans les exemples présentés la source d'énergie diélectrique est le micro-ondes mais pour des raisons de compatibilité (dégradabilité, réactivité diélectrique) à la formulation ou de nécessités industrielles (rapidité du procédé ou choix technologiques), ce mode d'apport énergétique peut être éventuellement et avantageusement remplacé par les hautes fréquences.

### Exemples n° 2

Exemple 2a : Une forme expansée microporeuse isotrope contenant 490 mg de maltodextrine (DE 19), 10 mg d'arôme orange et 100 mg de phloroglucinol dihydraté est obtenu après avoir soumis un mélange pâteux de viscosité voisine de 3000 mPa.s aux conditions expérimentales préalablement décrites (1b).

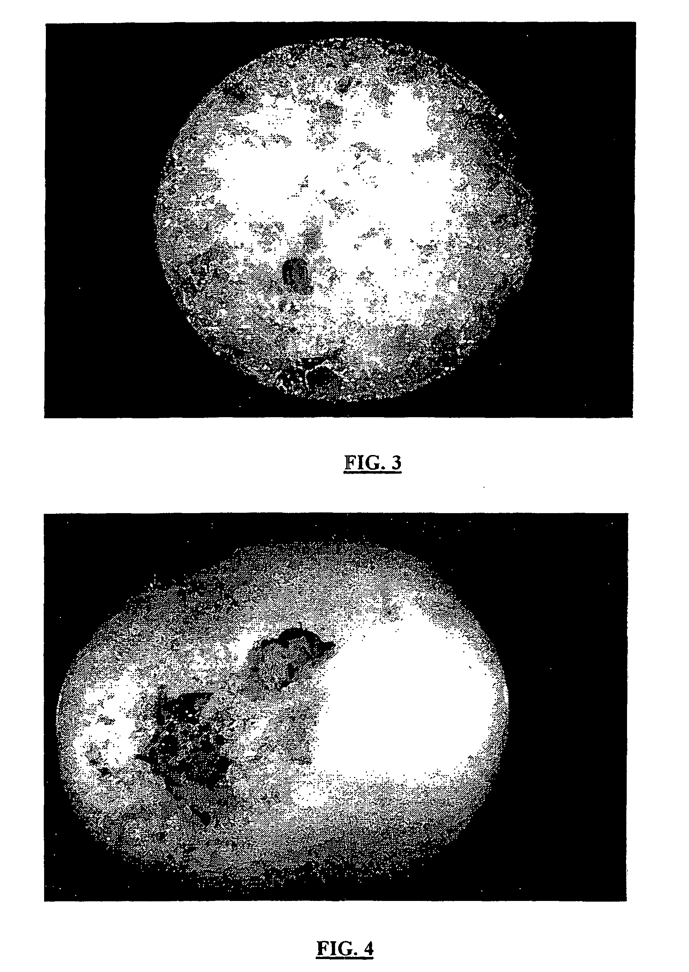

La forme expansée microporeuse isotrope obtenue de densité voisine de 0.21 et de volume 2.80 cm³ présente des caractéristiques de délitement et de forme en accord avec les objectifs (32 secondes) comme illustré sur la figure 3 (photographie avec facteur de grossissement de 5.5).

Exemple 2b : Une forme de même composition mais présentant un taux d'expansion non contrôlé ainsi qu'une structure expansée microporeuse très hétérogène est obtenue en soumettant le même mélange à des conditions de pression de 30.10² Pa (30 mbar) et une puissance absorbée de 4W. Cette forme bien qu'en accord avec l'objectif de délitement (30 secondes environ) n'est pas en accord avec les objectifs de forme vu l'irrégularité de la surface et du réseau interne obtenus.

### Exemple n° 3

Une forme expansée microporeuse isotrope contenant 588 mg de maltodextrine (DE 19), 10 mg d'arôme menthe et 100 mg de phloroglucinol est obtenue en soumettant un mélange de viscosité voisine de 3000 mPa.s aux conditions préalablement décrites (1b).

La forme expansée microporeuse isotrope présente un taux d'expansion contrôlé (volume final de 2.75 cm³ ) une densité voisine de 0.21 et se délite en environ 30 secondes dans 100 ml d'eau et de l'ordre d'une dizaine de seconde en bouche.

### Exemple n° 4

Une forme expansée microporeuse isotrope contenant 572 mg de maltodextrine (DE 19), 10 mg d'arôme menthe, 10 mg de xylitol et 100 mg de phloroglucinol est obtenu en soumettant un mélange de viscosité voisine de 3100 mPa.s aux conditions préalablement décrites (1b).

La forme expansée microporeuse isotrope obtenue en accord avec les objectifs présente un taux d'expansion (volume final de 2.95 cm³) une densité voisine de 0.22 et se délite en environ 32 secondes dans 100 ml d'eau et quasiment instantanément dans la bouche.

### Exemple n° 5

Une forme expansée microporeuse isotrope contenant 455 mg de maltodextrine (DE 19), 102 mg de PVP type Kollidon 12PF, 20 mg d'arôme menthe naturel, 20 mg de xylitol et 100 mg de phloroglucinol est obtenue en soumettant un mélange de viscosité voisine de 3000 mPa.s aux conditions préalablement décrites (1b).

La forme obtenue en accord avec les objectifs présente un taux d'expansion (volume final de 2.75 cm³ une densité voisine de 0.2, se délite en environ 30s dans 100 ml d'eau et instantanément au contact de la muqueuse buccale.

### Exemples 6

Exemple 6a : Une forme expansée microporeuse isotrope de composition suivante 515 mg de Maltodextrine (DE 19) et 85 mg de milnacipran est obtenue après avoir soumis au procédé un mélange de viscosité voisine de 2800 mPa.s dans les conditions décrites à l'exemple 1b.

Cette forme expansée microporeuse isotrope présente une densité voisine de 0.25 et se délite 30 secondes dans 100 ml d'eau et instantanément au contact de la muqueuse buccale.

Exemple 6b : Un mélange de même composition soumis aux mêmes conditions de puissance énergétique mais à des niveaux de pression inférieurs de l'ordre de 40. 10² Pa (40 mbar) présente une structure poreuse expansée de forme et de taille non contrôlées comme illustrée sur la photographie de la figure 4 avec un grossissement de 4 non compatible avec une utilisation dans le domaine pharmaceutique.

### Exemples 7

Exemple 7a : Une forme pharmaceutique expansée microporeuse isotrope de composition 515 mg de maltodextrine (DE 19), 85 mg de piroxicam est obtenue, après avoir introduit dans un blister polypropylène un mélange de viscosité voisine de 3500 mPa.s. Ce mélange est soumis dans le four micro-ondes sous vide aux conditions suivantes : 3.3 W absorbée par échantillon et un niveau de vide de 70.10² Pa (70 mbar) pendant 10 minutes.

Dans ces conditions expérimentales (7a), les échantillons présentent une structure conforme à l'objectif avec un taux d'expansion voisin de 3.5 et un délitement de 35 secondes dans 100 ml d'eau et instantanément au contact de la muqueuse buccale.

Exemple 7b : Dans des conditions expérimentales différentes à savoir de 8W absorbée par échantillon et un niveau de vide de 30.10² Pa (30 mbar) pendant 7 minutes, la forme obtenue de même composition bien que conforme aux objectifs en terme de délitement ne convient pas en terme de forme.

### Exemple n° 8

Une forme pharmaceutique expansée microporeuse isotrope de composition 515 mg de maltodextrine (DE 19), 85 mg de dompéridone en accord avec les objectifs selon l'invention est obtenue, après avoir introduit dans un blister polypropylène un mélange de viscosité voisine de 3500 mPa.s. Ce mélange est soumis dans le four micro-ondes sous vide aux conditions suivantes : 3W absorbée par échantillon et un niveau de vide de 65.10² Pa (65 mbar) pendant 10 min.

### Exemple n° 9

Une forme pharmaceutique expansée microporeuse isotrope de composition 100 mg de maltodextrine (DE 19), 650 mg de mannitol et 50 mg de piroxicam est obtenus après avoir soumis au procédé de séchage (entre 90. 10² et 500.10² Pa (90 et 500 mbar) pendant 0.5 h) une composition pâteuse de viscosité 2000 mPa.s. Dans ces conditions opératoires judicieusement choisies, la forme obtenue présente des caractéristiques morphologiques de délitement et en accord avec les objectifs.

### Exemple n° 10

Dans des conditions expérimentales décrites à l'exemple 1b, il a été possible d'obtenir des formes pharmaceutiques expansées microporeuses isotropes à délitement instantané de composition 100 mg de phloroglucinol, 40 mg de caséinate de sodium, 20 mg de xylitol et 400 mg de mannitol.

### Exemple n° 11

De façon similaire, des formes pharmaceutiques de la composition suivante à savoir 100 mg de phloroglucinol, 50 mg de chitosan et 400 mg de maltodextrine de DE voisin de 19 ont pu être obtenues. Ces formes présentent des caractéristiques morphologiques et de délitement en accord avec les objectifs.

### Exemple n° 12

Des mélanges à base uniquement de maltodextrine ou de sirop de glucose de degré Dextrose équivalent différents (6,14,21,34) aromatisés soit à l'aide d'arôme orange, menthe ou d'extrait de café et contenant initialement 30 à 40 % d'eau, ont permis après avoir été soumis aux micro-ondes sous vide (90.10² à 500.10² Pa (90 à 500 mbar) pendant 0.5 h) l'obtention de formes poreuses expansées, instantanément soluble dans l'eau et accord avec l'objectif au plan de la forme. Ces compositions monodoses expansées microporeuses isotropes peuvent être facilement utilisables comme boissons rafraîchissantes.

### Exemple 13

Des formes expansées microporeuses isotropes contenant 500 mg de lactose, 40 mg de Maltodextrine (DE 19) et 50 mg de piroxicam ont été obtenues en soumettant un mélange de teneur en eau initiale de l'ordre de 20 % (p/p) à une modulation des conditions expérimentales, en diminuant notamment la puissance micro-onde transmise à l'échantillon et en travaillant à des pressions comprises entre 100.10² et 500.10² (100 et 500 mbar) pendant 0,5 heure.

Ces formes présentent après exposition au traitement de l'invention une teneur en eau inférieur à 1% de la masse totale.

Ces formes expansées microporeuses isotropes présentent un temps de délitement en accord avec l'objectif.

### Exemple 14

Des formes expansées microporeuses isotropes contenant 500 mg de lactose, 30 mg de carboxyméthylcellulose sodique (basse viscosité) et 10 mg de piroxicam ont été obtenus en soumettant aux conditions expérimentales 13 un mélange de teneur en eau initiale de l'ordre de 30 % (p/p).

Ces formes présentent après exposition au traitement de l'invention une teneur en eau inférieur à 1% de la masse totale.

Ces formes expansées microporeuses isotropes présentent un temps de délitement en accord avec l'objectif

### Exemple 15

Des formes expansées microporeuses isotropes contenant 500 mg de lactose, 10 mg de gomme xanthane + 60 mg de maltodextrine de DE 34 et 10 mg de piroxicam ont été obtenues en soumettant aux conditions expérimentales 13 un mélange de teneur en eau initiale de l'ordre de 30 % (p/p)

Ces formes présentent après exposition au traitement de l'invention une teneur en eau inférieur à 1% de la masse totale.

Ces formes microporeuses présentent un temps de délitement en accord avec l'objectif.

### Exemple 16

Un lot de 500 formes expansées microporeuses contenant 450 mg de mannitol, 67 mg de maltodextrine de DE 19, 7 mg d'arôme menthe et 21 mg de piroxicam a été obtenu en 30 min sur un outil micro-ondes sous vide industriel dans des conditions similaires aux conditions opératoires préalablement décrites dans l'exemple 13.

Les formes obtenues présentant des caractéristiques morphologiques et de délitement en accord avec nos objectifs se sont en outre avérées stables après avoir été soumises à une étude de stabilité accélérée à 40°C/75 % d'Humidité Relative pendant 6 mois.

### Exemple 17

Un lot de 500 formes expansées microporeuses contenant 450 mg de mannitol, 67 mg de maltodextrine de DE 19, 7 mg d'arôme menthe et 21 mg de dompéridone a été obtenu en 30 minutes sur un outil micro-ondes sous vide industriel dans des conditions similaires aux conditions opératoires préalablement décrites dans l'exemple 13.

Les formes obtenues présentant des caractéristiques morphologiques et de délitement en accord avec nos objectifs se sont en outre avérées stables après avoir été soumises à une étude de stabilité accélérée à 40°C/75 % d'Humidité Relative pendant 6 mois.

## Revendications

1. Composition pour une utilisation pharmaceutique, vétérinaire, alimentaire, diététique ou cosmétique, comprenant 1 % à 50 % en poids d'un ou plusieurs principe(s) actif(s), 50 % à 99 % en poids d'un support comprenant un ou plusieurs polymères, éventuellement un ou plusieurs diluant(s) et éventuellement un ou plusieurs additif(s), notamment arôme ou colorant, ladite composition étant **caractérisée en ce qu'**elle présente une structure expansée microporeuse isotrope, les polymères étant choisis dans le groupe constitué par les polymères d'origine végétale, éventuellement en combinaison avec les polymères d'origine animale ou les polymères de synthèse, et ledit support étant tel que le ou les polymère(s) sont présents dans la composition dans une proportion supérieure ou égale à 1% (p/p) et plus particulièrement comprise entre 6 % et 98 % (p/p) et **en ce qu'**elle est susceptible d'être obtenue par le procédé comprenant les étapes de :
- homogénéisation d'une formulation pâteuse comprenant le ou les principes actifs, le ou les polymères, éventuellement le ou les additifs et le ou les diluants,
- injection dans un élément de moulage,
- séchage et moulage simultanés par un procédé type micro-ondes ou hautes fréquences avec un niveau de vide compris entre 30 et 700.10² Pa.

2. Composition pour une utilisation pharmaceutique, vétérinaire, alimentaire, diététique ou cosmétique selon la revendication 1, **caractérisée en ce que** le polymère d'origine végétale est choisi parmi les polysaccharides obtenus par hydrolyse chimique ou enzymatique de l'amidon modifié chimiquement, les polymères de type cellulosique chimiquement modifiés ou les polymères de type gomme ou leurs mélanges.

3. Composition pour une utilisation pharmaceutique, vétérinaire, alimentaire, diététique ou cosmétique selon la revendication 2, **caractérisée en ce que** le polysaccharide est choisi parmi les maltodextrines ou sirops de glucose, les glycolates sodique d'amidon ou leurs mélanges.

4. Composition pour une utilisation pharmaceutique, vétérinaire, alimentaire, diététique ou cosmétique selon la revendication 3, **caractérisée en ce que** le polymère d'origine végétale est choisi parmi les maltodextrines ou sirops de glucose de degrés dextrose équivalent (DE) compris entre 3 et 50 et préférentiellement entre 6 et 34 ou leurs mélanges.

5. Composition pour une utilisation pharmaceutique, vétérinaire, alimentaire, diététique ou cosmétique microporeuse expansée isotrope selon la revendication 2, **caractérisée en ce que** le polymère d'origine végétale de type cellulosique est choisi parmi la carboxyméthylcellulose sodique basse ou moyenne viscosité, l'hydroxypropylméthylcellulose, l'hydroxypropylcellulose, l'hydroxyéthylcellulose ou leurs mélanges.

6. Composition pour une utilisation pharmaceutique, vétérinaire, alimentaire, diététique ou cosmétique microporeuse expansée isotrope selon la revendication 2, **caractérisée en ce que** le polymère d'origine végétale est de type gomme guar, arabique, xanthane, pectine et alginates ou leurs mélanges.

7. Composition pour une utilisation pharmaceutique, vétérinaire, alimentaire, diététique ou cosmétique selon la revendication 1, **caractérisée en ce que** le polymère de synthèse est la polyvinylpyrrolidone.

8. Composition pour une utilisation pharmaceutique, vétérinaire, alimentaire, diététique ou cosmétique selon la revendication 1, **caractérisée en ce que** le polymère d'origine animale est choisi parmi les caséinates sodiques, le chitosan et leurs dérivés d'hydrolyse solubles à l'eau, la gélatine, le collagène, l'acide chondroîtique sulfate et leurs hydrolysats ou leurs mélanges.

9. Composition pour une utilisation pharmaceutique, vétérinaire, alimentaire, diététique ou cosmétique microporeuse expansée isotrope selon l'une des revendications précédentes, **caractérisée en ce que** ledit ou lesdits polymères sont présents dans la formulation à un pourcentage au moins égal à 1% (p/p) et plus particulièrement entre 6 % et 98% (p/p), et compatible avec une viscosité comprise entre 100 mPa.s et 100 000 mPa.s.

10. Composition pour une utilisation pharmaceutique, vétérinaire, alimentaire, diététique ou cosmétique microporeuse expansée isotrope selon la revendication 9, **caractérisée en ce que** ledit ou lesdits polymères sont présents dans la formulation à un pourcentage au moins égal à 1 % (p/p) et plus particulièrement entre 6 et 98 % (p/p), et compatible avec une viscosité comprise entre 100 et 50 000 mPa.s.

11. Composition pour une utilisation pharmaceutique, vétérinaire, alimentaire, diététique ou cosmétique microporeuse expansée isotrope selon l'une des revendications précédentes, **caractérisée en ce que** le diluant optionnel est choisi parmi le mannitol, le saccharose, le lactose, le fructose, le sorbitol, le xylitol, le maltitol et le phosphate dicalcique dihydraté.

12. Composition pour une utilisation pharmaceutique, vétérinaire, alimentaire, diététique ou cosmétique microporeuse expansée isotrope selon l'une des revendications précédentes, **caractérisée en ce que** la densité est inférieure à 0.9 g/cm³.

13. Composition pour une utilisation pharmaceutique, vétérinaire, alimentaire, diététique ou cosmétique microporeuse expansée isotrope selon la revendication 12, **caractérisée en ce que** la densité est comprise entre 0.2 et 0.7 g/cm³.

14. Composition pour une utilisation pharmaceutique, vétérinaire, alimentaire, diététique ou cosmétique microporeuse expansée isotrope selon l'une des revendications précédentes, **caractérisée en ce que** le ou les principes actifs dans la matrice microporeuse expansée isotrope sont à l'état dissous ou dispersés ou sous formes pelliculées.

15. Composition pour une utilisation pharmaceutique, vétérinaire, alimentaire, diététique ou cosmétique microporeuse expansée isotrope selon la revendication 14, **caractérisée en ce que** le ou les principes actifs sont choisis, à titre non limitatif, parmi les analgésiques, les anti-migraineux, les analgésiques antipyrétiques et/ou anti-inflammatoires, les anesthésiques locaux, les anti-angineux, les antispasmodiques anti-cholinergiques, les anti-secrétoires, les myorelaxants, les antinauséeux, les vasodilatateurs centraux et périphériques.

16. Composition pour une utilisation pharmaceutique, vétérinaire, alimentaire, diététique ou cosmétique microporeuse expansée isotrope selon la revendication 15, **caractérisée en ce que** le principe actif est choisi dans le groupe constitué par le Milnacipran, le piroxicam, le phloroglucinol, la dompéridone.

17. Composition pour une utilisation pharmaceutique, vétérinaire, alimentaire, diététique ou cosmétique microporeuse expansée isotrope selon l'une des revendications précédentes, **caractérisée en ce que** le conditionnement final servant d'élément de moulage est de type polypropylène.

18. Composition pour une utilisation pharmaceutique, vétérinaire, alimentaire, diététique ou cosmétique microporeuse expansée isotrope selon l'une des revendications 1 à 16, **caractérisée en ce que** le conditionnement final est de type polytétrafluoroéthylène (ex : Téflon® ).

19. Procédé de préparation d'une composition à usage pharmaceutique, vétérinaire, alimentaire, diététique ou cosmétique selon l'une des revendications 1 à 18, **caractérisé en ce que** l'on homogénéise une formulation pâteuse comprenant un ou plusieurs principes actifs, un ou plusieurs polymères, éventuellement un ou plusieurs additifs et un ou plusieurs diluants, on l'injecte dans un élément de moulage, puis **en ce que** l'on sèche et moule simultanément par un procédé de type micro-ondes ou hautes fréquences avec un niveau de vide compris entre 30 et 700.10² Pa et préférentiellement compris entre 60 et 500.10² Pa (30 et 700 mbar et préférentiellement compris entre 60 et 500 mbar) pour donner naissance à une structure expansée microporeuse isotrope de forme régulière, notamment de masse volumique inférieure à 0,9 g/cm³.

20. Procédé de préparation d'une composition à usage pharmaceutique, vétérinaire, alimentaire, diététique ou cosmétique microporeuse expansée isotrope selon la revendication 19, **caractérisé en ce que** la formulation pâteuse obtenue par homogénéisation présente une viscosité comprise entre 100 mPa.s et 100 000 mPa.s, préférentiellement entre 100 et 50 000 mPa.s, puis injection ou extrusion de cette masse dans le conditionnement final.

21. Procédé de préparation d'une composition à usage pharmaceutique, vétérinaire, alimentaire, diététique ou cosmétique microporeuse expansée isotrope selon l'une des revendications 19 ou 20, **caractérisé en ce que** les températures pendant la phase de séchage et mise en forme sont comprises entre 25 °C et 80 °C évitant de ce fait la dégradation des principes actifs thermolabiles.

22. Procédé de préparation d'une composition à usage pharmaceutique, vétérinaire, alimentaire, diététique ou cosmétique microporeuse expansée isotrope selon l'une des revendications 19 à 21, **caractérisé en ce que** la durée des opérations de séchage et mise en forme sont simultanées et sont inférieures à 1 heure préférentiellement 30 minutes.

23. Procédé de préparation d'une composition à usage pharmaceutique, vétérinaire, alimentaire, diététique ou cosmétique microporeuse expansée isotrope selon l'une des revendications 19 à 22, **caractérisé en ce que** l'élément dans lequel s'effectue le séchage-moulage simultané est le conditionnement final.

24. Procédé de préparation d'une composition à usage pharmaceutique, vétérinaire, alimentaire, diététique ou cosmétique microporeuse expansée isotrope, selon l'une des revendications 19 à 23, **caractérisé en ce que** le procédé d'obtention est effectué en continu.

25. Utilisation d'une composition selon l'une quelconque des revendications 1 à 18 en tant que composition à dissolution rapide.

26. Utilisation selon la revendication 25, **caractérisé en ce que** la composition présente un temps de délitement inférieur à 1 minute, préférentiellement 30 secondes, dans les conditions d'utilisation au contact direct d'une muqueuse notamment d'une muqueuse buccale ou dans un volume adapté d'eau.

## Patentansprüche

1. Zusammensetzung zur pharmazeutischen, veterinärmedizinischen, Nahrungsmittel-, diätetischen oder kosmetischen Verwendung, umfassend 1 bis 50 Gewichts-% eines oder mehrerer Wirkstoffe, 50 bis 99 Gewichts-% eines Trägers, der ein oder mehrere Polymere umfasst, gegebenenfalls ein oder mehrere Verdünnungsmittel und gegebenenfalls einen oder mehrere Zusätze, insbesondere Aroma oder Farbstoff, wobei die Zusammensetzung **dadurch gekennzeichnet ist, dass** sie eine geschäumte mikroporöse isotrope Struktur aufweist, wobei die Polymere aus der Gruppe ausgewählt sind, die aus Polymeren pflanzlichen Ursprungs, gegebenenfalls in Kombination mit Polymeren tierischen Ursprungs oder synthetischen Polymeren, besteht, und der Träger derart ist, dass das oder die Polymer(e) in der Zusammensetzung in einem Verhältnis größer oder gleich 1% (Gew./Gew.) und spezieller zwischen 6 und 98% (GewJGew.) eingeschlossen vorliegen, und dadurch, dass sie durch das Verfahren erhältlich ist, welches die Schritte umfasst:
- Homogenisierung einer dickflüssigen Formulierung, die den einen oder die mehreren Wirkstoff(e), das oder die Polymer(e), gegebenenfalls den Zusatz oder die Zusätze und das oder die Verdünnungsmittel umfasst,
- Einspritzen in ein Formungselement,
- gleichzeitiges Trocknen und Formen durch ein Verfahren der Mikrowellenoder Hochfrequenz-Art mit einem Vakuumniveau von 30 bis 700·10² Pa.

2. Zusammensetzung zur pharmazeutischen, veterinärmedizinischen, Nahrungsmittel-, diätetischen oder kosmetischen Verwendung nach Anspruch 1, **dadurch gekennzeichnet, dass** das Polymer pflanzlichen Ursprungs aus Polysacchariden, die durch chemische oder enzymatische Hydrolyse von chemisch modifizierter Stärke erhalten werden, Polymeren der chemisch modifizierten Cellulose-Derivat-Art oder Polymeren der Pflanzengummi-Art oder deren Mischungen ausgewählt ist.

3. Zusammensetzung zur pharmazeutischen, veterinärmedizinischen, Nahrungsmittel-, diätetischen oder kosmetischen Verwendung nach Anspruch 2, **dadurch gekennzeichnet, dass** das Polysaccharid aus Maltodextrinen oder Glucosesirupen, Stärke-Natriumglycolaten oder deren Mischungen ausgewählt ist.

4. Zusammensetzung zur pharmazeutischen, veterinärmedizinischen, Nahrungsmittel-, diätetischen oder kosmetischen Verwendung nach Anspruch 3, **dadurch gekennzeichnet, dass** das Polymer pflanzlichen Ursprungs aus Maltodextrinen oder Glucosesirupen mit Dextrose-Äquivalent (DE)-Graden, die zwischen 3 und 50 und vorzugsweise zwischen 6 und 34 eingeschlossen sind, oder deren Mischungen ausgewählt ist.

5. Mikroporöse geschäumte isotrope Zusammensetzung zur pharmazeutischen, veterinärmedizinischen, Nahrungsmittel-, diätetischen oder kosmetischen Verwendung nach Anspruch 2, **dadurch gekennzeichnet, dass** das Polymer pflanzlichen Ursprungs der Cellulose-Derivat-Art aus Natriumcarboxymethylcellulose mit niedriger oder mittlerer Viskosität, Hydroxypropylmethylcellulose, Hydroxypropylcellulose, Hydroxyethylcellulose oder deren Mischungen ausgewählt ist.

6. Mikroporöse geschäumte isotrope Zusammensetzung zur pharmazeutischen, veterinärmedizinischen, Nahrungsmittel-, diätetischen oder kosmetischen Verwendung nach Anspruch 2, **dadurch gekennzeichnet, dass** es sich bei dem Polymer pflanzlichen Ursprungs um Guargummi, Gummi arabicum, Xanthangummi, Pectin und Alginate oder deren Mischungen handelt.

7. Zusammensetzung zur pharmazeutischen, veterinärmedizinischen, Nahrungsmittel-, diätetischen oder kosmetischen Verwendung nach Anspruch 1, **dadurch gekennzeichnet, dass** das synthetische Polymer Polyvinylpyrrolidon ist.

8. Zusammensetzung zur pharmazeutischen, veterinärmedizinischen, Nahrungsmittel-, diätetischen oder kosmetischen Verwendung nach Anspruch 1, **dadurch gekennzeichnet, dass** das Polymer tierischen Ursprungs aus Natriumcaseinaten, Chitosan und dessen in Wasser löslichen Hydrolyse-Derivaten, Gelatine, Kollagen, Chondroitinsäuresulfat und deren Hydrolysaten oder deren Mischungen ausgewählt ist.

9. Mikroporöse geschäumte isotrope Zusammensetzung zur pharmazeutischen, veterinärmedizinischen, Nahrungsmittel-, diätetischen oder kosmetischen Verwendung nach einem der vorangehenden Ansprüche, **dadurch gekennzeichnet, dass** das oder die Polymer(e) in der Formulierung zu einem Prozentsatz von mindestens gleich 1% (Gew./Gew.) und spezieller zwischen 6% und 98% (Gew./Gew.) vorliegen und mit einer Viskosität zwischen 100 mPa.s und 100000 mPa.s einschließlich kompatibel sind.

10. Mikroporöse geschäumte isotrope Zusammensetzung zur pharmazeutischen, veterinärmedizinischen, Nahrungsmittel-, diätetischen oder kosmetischen Verwendung nach Anspruch 9, **dadurch gekennzeichnet, dass** das oder die Polymer(e) in der Formulierung zu einem Prozentsatz von mindestens gleich 1% (Gew./Gew.) und spezieller zwischen 6 und 98% (Gew./Gew.) vorliegen und mit einer Viskosität zwischen 100 und 50000 mPa.s einschließlich kompatibel sind.

11. Mikroporöse geschäumte isotrope Zusammensetzung zur pharmazeutischen, veterinärmedizinischen, Nahrungsmittel-, diätetischen oder kosmetischen Verwendung nach einem der vorangehenden Ansprüche, **dadurch gekennzeichnet, dass** das fakultative Verdünnungsmittel aus Mannit, Saccharose, Lactose, Fructose, Sorbit, Xylit, Maltit und Dicalciumphosphatdihydrat ausgewählt ist.

12. Mikroporöse geschäumte isotrope Zusammensetzung zur pharmazeutischen, veterinärmedizinischen, Nahrungsmittel-, diätetischen oder kosmetischen Verwendung nach einem der vorangehenden Ansprüche, **dadurch gekennzeichnet, dass** die Dichte unterhalb von 0,9 g/cm³ liegt.

13. Mikroporöse geschäumte isotrope Zusammensetzung zur pharmazeutischen, veterinärmedizinischen, Nahrungsmittel-, diätetischen oder kosmetischen Verwendung nach Anspruch 12, **dadurch gekennzeichnet, dass** die Dichte zwischen 0,2 und 0,7 g/cm³ einschließlich liegt.

14. Mikroporöse geschäumte isotrope Zusammensetzung zur pharmazeutischen, veterinärmedizinischen, Nahrungsmittel-, diätetischen oder kosmetischen Verwendung nach einem der vorangehenden Ansprüche, **dadurch gekennzeichnet, dass** der oder die Wirkstoff(e) in der mikroporösen geschäumten isotropen Matrix in gelöstem oder dispergiertem Zustand oder in schuppenartigen Formen vorliegen.

15. Mikroporöse geschäumte isotrope Zusammensetzung zur pharmazeutischen, veterinärmedizinischen, Nahrungsmittel-, diätetischen oder kosmetischen Verwendung nach Anspruch 14, **dadurch gekennzeichnet, dass** der oder die Wirkstoff(e) ohne Beschränkung aus Analgetika, Antimigränemitteln, antipyretischen und/oder entzündungshemmenden Analgetika, Lokalanästhetika, Antianginamitteln, spasmolytischen Anticholinergika, sekretionshemmenden Mitteln, Muskelrelaxantien, gegen Nausea wirkenden Mitteln, zentralen und peripheren Vasodilatatoren ausgewählt sind.

16. Mikroporöse geschäumte isotrope Zusammensetzung zur pharmazeutischen, veterinärmedizinischen, Nahrungsmittel-, diätetischen oder kosmetischen Verwendung nach Anspruch 15, **dadurch gekennzeichnet, dass** der Wirkstoff aus der Gruppe ausgewählt ist, die aus Milnacipran, Piroxicam, Phloroglucinol, Domperidon besteht.

17. Mikroporöse geschäumte isotrope Zusammensetzung zur pharmazeutischen, veterinärmedizinischen, Nahrungsmittel-, diätetischen oder kosmetischen Verwendung nach einem der vorangehenden Ansprüche,
**dadurch gekennzeichnet, dass** die Endverpackung als Formungselement dient und vom Polypropylen-Typ ist.

18. Mikroporöse geschäumte isotrope Zusammensetzung zur pharmazeutischen, veterinärmedizinischen, Nahrungsmittel-, diätetischen oder kosmetischen Verwendung nach einem der Ansprüche 1 bis 16, **dadurch gekennzeichnet, dass** die Endverpackung vom Polytetrafluorethylen- (Bsp.: Teflon® )Typ ist.

19. Verfahren zur Herstellung einer Zusammensetzung zur pharmazeutischen, veterinärmedizinischen, Nahrungsmittel-, diätetischen oder kosmetischen Verwendung nach einem der Ansprüche 1 bis 18, **dadurch gekennzeichnet, dass** man eine dickflüssige Formulierung, die einen oder mehrere Wirkstoffe, ein oder mehrere Polymere, gegebenenfalls einen oder mehrere Zusätze und ein oder mehrere Verdünnungsmittel umfasst, homogenisiert, sie in ein Formungselement einspritzt, dann dadurch, dass man gleichzeitig durch ein Verfahren der Mikrowellen- oder Hochfrequenz-Art mit einem Vakuumniveau, das zwischen 30 und 700·10² Pa einschließlich, und vorzugsweise zwischen 60 und 500·10² Pa einschließlich (30 und 700 mBar und vorzugsweise zwischen 60 und 500 mBar einschließlich) trocknet und formt, um eine geschäumte mikroporöse isotrope Struktur mit regelmäßiger Form, insbesondere mit einer Dichte unterhalb von 0,9 g/cm³, entstehen zu lassen.

20. Verfahren zur Herstellung einer mikroporösen geschäumten isotropen Zusammensetzung zur pharmazeutischen, veterinärmedizinischen, Nahrungsmittel-, diätetischen oder kosmetischen Verwendung nach Anspruch 19, **dadurch gekennzeichnet, dass** die dickflüssige Formulierung, die durch Homogenisierung erhalten wird, eine Viskosität zwischen 100 mPa.s und 100000 mPa.s einschließlich, vorzugsweise zwischen 100 und 50000 mPa.s aufweist, dann Einspritzen oder Extrusion dieser Masse in die Endverpackung.

21. Verfahren zur Herstellung einer mikroporösen geschäumten isotropen Zusammensetzung zur pharmazeutischen, veterinärmedizinischen, Nahrungsmittel-, diätetischen oder kosmetischen Verwendung nach einem der Ansprüche 19 oder 20, **dadurch gekennzeichnet, dass** die Temperaturen während der Trocknungs- und Formungsphase zwischen 25°C und 80°C einschließlich liegen, wodurch man die Zersetzung von wärmelabilen Wirkstoffen vermeidet.

22. Verfahren zur Herstellung einer mikroporösen geschäumten isotropen Zusammensetzung zur pharmazeutischen, veterinärmedizinischen, Nahrungsmittel-, diätetischen oder kosmetischen Verwendung nach einem der Ansprüche 19 bis 21, **dadurch gekennzeichnet, dass** die Dauer des Trocknungs- und Formungsvorgangs simultan ist und unterhalb einer Stunde, bevorzugt 30 Minuten liegt.

23. Verfahren zur Herstellung einer mikroporösen geschäumten isotropen Zusammensetzung zur pharmazeutischen, veterinärmedizinischen, Nahrungsmittel-, diätetischen oder kosmetischen Verwendung nach einem der Ansprüche 19 bis 22, **dadurch gekennzeichnet, dass** das Element, in dem die gleichzeitige Trocknung-Formung bewirkt wird, die Endverpackung ist.

24. Verfahren zur Herstellung einer mikroporösen geschäumten isotropen Zusammensetzung zur pharmazeutischen, veterinärmedizinischen, Nahrungsmittel-, diätetischen oder kosmetischen Verwendung nach einem der Ansprüche 19 bis 23, **dadurch gekennzeichnet, dass** das Verfahren zum Erhalt kontinuierlich durchgeführt wird.

25. Verwendung einer Zusammensetzung nach irgendeinem der Ansprüche 1 bis 18 als Zusammensetzung mit rascher Auflösung.

26. Verwendung nach Anspruch 25, **dadurch gekennzeichnet, dass** die Zusammensetzung eine Zeit des Zerfalls unterhalb von einer Minute, vorzugsweise 30 Sekunden bei den Verwendungsbedingungen in direktem Kontakt mit einer Schleimhaut, insbesondere einer bukkalen Schleimhaut, oder in einem passenden Wasservolumen aufweist.

## Claims

1. Composition for pharmaceutical, veterinary, food, dietetic or cosmetic use, comprising 1% to 50% by weight of one or more active ingredients, 50% to 99% by weight of a carrier comprising one or more polymers, optionally one or more diluents and optionally one or more additives, in particular a flavouring or a colouring, said composition being **characterized in that** it has an isotropic microporous expanded structure and the polymers being chosen from the group consisting of polymers of plant origin, optionally in combination with polymers of animal origin or synthetic polymers, and said carrier being such that the polymer (s) are present in the composition in a proportion greater than or equal to 1% (w/w) and more particularly of between 6% and 98% and **in that** it is obtainable by the method comprising the steps of:
- homogenizing a pasty formulation comprising the active ingredient (s), the polymer(s), optionally the additive(s) and the diluent(s),
- injecting into a moulding component,
- simultaneous drying and moulding by a microwave or high-frequency type method with a vacuum level of between 30 and 700 × 10² Pa.

2. Composition for pharmaceutical, veterinary, food, dietetic or cosmetic use according to Claim 1, **characterized in that** the polymer of plant origin is chosen from the polysaccharides obtained by chemical or enzymatic hydrolysis of the chemically modified starch, the polymers of the chemically modified cellulosic type or the polymers of the gum type or mixtures thereof.

3. Composition for pharmaceutical, veterinary, food, dietetic or cosmetic use according to Claim 2, **characterized in that** the polysaccharide is chosen from maltodextrins or glucose syrups, and sodium glycolates of starch or mixtures thereof.

4. Composition for pharmaceutical, veterinary, food, dietetic or cosmetic use according to Claim 3, **characterized in that** the polymer of plant origin is chosen from maltodextrins and glucose syrups having a dextrose equivalent (DE) level of between 3 and 50 and preferably between 6 and 34 or mixtures thereof.

5. Isotropic expanded microporous composition for pharmaceutical, veterinary, food, dietetic or cosmetic use according to Claim 2, **characterized in that** the polymer of plant origin of the cellulosic type is chosen from carboxymethyl cellulose sodium low or medium viscosity, hydroxypropyl methyl cellulose, hydroxypropyl cellulose, hydroxyethyl cellulose or mixtures thereof.

6. Isotropic expanded microporous composition for pharmaceutical, veterinary, food, dietetic or cosmetic use according to Claim 2, **characterized in that** the polymer of plant origin is of the guar gum, gum arabic, xanthane, pectin and alginate type or mixtures thereof.

7. Composition for pharmaceutical, veterinary, food, dietetic or cosmetic use according to Claim 1, **characterized in that** the synthetic polymer is polyvinylpyrrolidone.

8. Composition for pharmaceutical, veterinary, food, dietetic or cosmetic use according to Claim 1, **characterized in that** the polymer of animal origin is chosen from sodium caseinates, chitosan, their water-soluble hydrolysis derivatives, gelatin, collagen, chondroitic acid sulphate and hydrolysates thereof or mixtures thereof.

9. Isotropic expanded microporous composition for pharmaceutical, veterinary, food, dietetic or cosmetic use according to any one of the preceding claims, **characterized in that** said polymer(s) is/are present in the formulation at a percentage at least equal to 1% (w/w) and more particularly between 6% and 98% (w/w), and compatible with a viscosity of between 100 mPa.s and 100,000 mPa.s.

10. Isotropic expanded microporous composition for pharmaceutical, veterinary, food, dietetic or cosmetic use according to Claim 9, **characterized in that** said polymer(s) are present in the formulation at a percentage at least equal to 1% (w/w) and more particularly between 6 and 98% (w/w), and compatible with a viscosity of between 100 and 50,000 mPa.s.

11. Isotropic expanded microporous composition for pharmaceutical, veterinary, food, dietetic or cosmetic use according to one of the preceding claims, **characterized in that** the optional diluent is chosen from mannitol, sucrose, lactose, fructose, sorbitol, xylitol, maltitol and dicalcium phosphate dihydrate.

12. Isotropic expanded microporous composition for pharmaceutical, veterinary, food, dietetic or cosmetic use according to one of the preceding claims, **characterized in that** the density is less than 0.9 g/cm³.

13. Isotropic expanded microporous composition for pharmaceutical, veterinary, food, dietetic or cosmetic use according to Claim 12, **characterized in that** the density is between 0.2 and 0.7 g/cm³.

14. Isotropic expanded microporous composition for pharmaceutical, veterinary, food, dietetic or cosmetic use according to one of the preceding claims, **characterized in that** the active ingredient(s) in the isotropic expanded microporous matrix are in the dissolved or dispersed state or in film-coated forms.

15. Isotropic expanded microporous composition for pharmaceutical, veterinary, food, dietetic or cosmetic use according to Claim 14, **characterized in that** the active ingredient(s) are chosen, without limitation, from analgesics, antimigraines, antipyretic and/or antiinflammatory. analgesics agents, local anaesthetics, antianginals, anticholinergic antispasmodics, antisecretory agents, muscle relaxants, antinauseants, central and peripheral vasodilators.

16. Isotropic expanded microporous composition for pharmaceutical, veterinary, food, dietetic or cosmetic use according to Claim 15, **characterized in that** the active ingredient is chosen from the group consisting of Milnacipran, piroxicam, phloroglucinol, domperidone.

17. Isotropic expanded microporous composition for pharmaceutical, veterinary, food, dietetic or cosmetic use according to one of the preceding claims, **characterized in that** the final packaging serving as moulding component is of the polypropylene type.

18. Isotropic expanded microporous composition for pharmaceutical, veterinary, food, dietetic or cosmetic use according to one of Claims 1 to 16, **characterized in that** the final packaging is of the polytetrafluoroethylene type (e.g.: Teflon@).

19. Method for preparing a composition for pharmaceutical, veterinary, food, dietetic or cosmetic use according to Claims 1 to 18, **characterized in that** a pasty formulation comprising one or more active ingredients, one or more polymers, optionally one or more additives and one or more diluents is homogenized, it is injected into a moulding component, and then **in that** drying and moulding are carried out simultaneously by a microwave or high frequency type process with a vacuum level of between 30 and 700 × 10² Pa and preferably between 60 and 500 × 10² Pa (30 and 700 mbar and preferably between 60 and 500 mbar) to give rise to an isotropic microporous expanded structure of regular form, in particular having a density of less than 0.9 g/cm³.

20. Method for preparing an isotropic expanded microporous composition for pharmaceutical, veterinary, food, dietetic or cosmetic use according to Claim 19, **characterized in that** the pasty formulation obtained by homogenization has a viscosity of between 100 mPa.s and 100,000 mPa.s, preferably between 100 and 50,000 mPa.s, followed by injection or extrusion of this mass into the final packaging.

21. Method for preparing an isotropic expanded microporous composition for pharmaceutical, veterinary, food, dietetic or cosmetic use according to either of Claims 19 and 20, **characterized in that** the temperatures during the drying and forming phase are between 25°C and 80°C, thereby avoiding the degradation of the heat-labile active ingredients.

22. Method for preparing an isotropic expanded microporous composition for pharmaceutical, veterinary, food, dietetic or cosmetic use according to Claims 19 to 21, **characterized in that** the duration of the drying and forming operations are simultaneous and are less than 1 hour, preferably 30 minutes.

23. Method for preparing an isotropic expanded microporous composition for pharmaceutical, veterinary, food, dietetic or cosmetic use according to one of Claims 19 to 22, **characterized in that** the component in which simultaneous drying-moulding is carried out is the final packaging.

24. Method for preparing an isotropic expanded microporous composition for pharmaceutical, veterinary, food, dietetic or cosmetic use according to one of Claims 19 to 23, **characterized in that** the method of production is carried out continuously.

25. Use of a composition according to any one of Claims 1 to 18, as a fast-dissolving composition.

26. Use according to Claim 25, **characterized in that** the composition has a disintegration time of less than 1 minute, preferably 30 seconds, under conditions of use on direct contact with a mucous membrane, in particular the buccal mucous membrane or in an appropriate volume of water.
